# EUROPÄISCHE PATENTSCHRIFT

(11) **EP 1 536 397 B1**
(45) Veröffentlichungstag und Bekanntmachung des Hinweises auf die Patenterteilung: **07.03.2018**
(21) Anmeldenummer: 04101672.6
(22) Anmeldetag: 22.04.2004
(51) Int. Cl.: G09F 3/02, G09F 3/10, B65C 3/06

(54) **Etikett, Spritzenkörper und Spritzenanordnung mit Etikett**
Label, syringe and injection system with label
Etiquette,seringue et système d'injection avec étiquette

(30) Priorität: 26.11.2003 EP 03104390
(43) Veröffentlichungstag der Anmeldung: 01.06.2005
(73) Patentinhaber: Schreiner Group GmbH & Co. KG, 85764 Oberschleissheim (DE)
(72) Erfinder: Seidl, Peter, 81371, München (DE); Moosheimer, Ulrich, 85411, Hohenkammern (DE)
(74) Vertreter: Epping - Hermann - Fischer

(56) Entgegenhaltungen:
- EP-A1- 0 463 193
- EP-A1- 1 044 698
- EP-A2- 0 935 968
- US-A- 4 312 523
- US-A- 5 692 640
- US-B1- 6 193 279
- US-B1- 6 332 631

## Beschreibung

Die vorliegende Erfindung betrifft eine Spritzenanordnung mit einem Spritzenkörper, welcher ein streifenförmiges Etikett, insbesondere ein Etikett zur Überrundum-Etikettierung des Spritzenkörpers aufweist.

Etiketten der eingangs genannten Art sind häufig so gestaltet, daß ihre Ausdehnung in Streifenlängsrichtung größer ist, als der Durchmesser des Spritzenkörpers oder sonstigen Gefäßes, und sie folglich selbstüberlappend aufgeklebt werden. Eine entsprechende Gestaltung wird insbesondere dann gewählt, wenn die Spritzen, Ampullen und Phiolen für Pharmazeutika aufgrund ihrer geringen Größe sonst zu wenig Mantelfläche böten, um alle zur Kennzeichung des Inhalts erforderlichen Informationen auf einem nichtüberlappenden Etikett unterzubringen.

Der Platzbedarf steigt, wenn das Etikett mit sogenannten Belegabschnitten ausgestattet ist. Darunter versteht man vom restlichen Etikett trennbare Bereiche, welche einen Teil der Etiketteninformation tragen (welche in der Regel nochmalig auf den am Behältnis verbliebenen Teil des Etiketts aufgedruckt ist) und separat auf ein Dokumentationsblatt oder dergleichen aufgeklebt werden können. Sie können beispielsweise dazu dienen festzuhalten, welche Dosis von welchem Medikament ein Patient in welcher Abfolge erhalten hat. Ein derartiges Etikett ist beispielsweise aus EP 0 463 193 A1, US 6,193,279 B1 und aus US 4,312,523 A bekannt. In der US 5,692,640 A ist eine Verspendeeinrichtung zum Verspenden von Etiketten, die auf eine Arzneimittelflasche angeordnet werden kann, beschrieben.

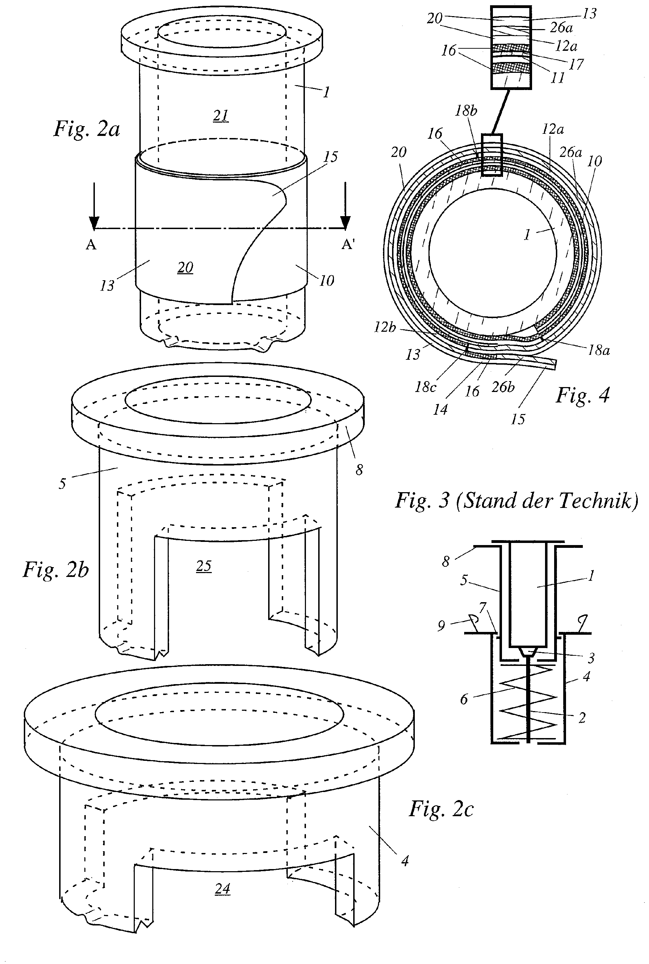

Sind Spritzenkörper, worunter der das Injektat enthaltende Teil einer Spritze verstanden wird, an welchen eine Kanüle angesteckt oder angeschraubt wird, mit selbstüberlappenden, Belegabschnitte enthaltenden Etiketten ausgestattet, so ergibt sich oft ein spezielles Handhabungsproblem, welches aus der Verwendung sogenannter Spritzenprotektoren resultiert. Spritzenprotektoren dienen dazu, eine Spritze vor deren Anwendung so zu verwahren, daß zum einen ein Schutz gegen Verletzung an der Spitze der Kanüle besteht, zum anderen die Kanüle selbst gegen Brechen oder Verbiegen geschützt ist. Der grundsätzliche Aufbau einer Spritzenanordnung mit Spritzenprotektor ist nachfolgend kurz anhand der in Fig. 3 abgebildeten, schematischen Schnittdarstellung erläutert.

Der Spritzenkörper 1, an welchen die Kanüle 2 mittels eines Verbindungsstücks 3 angesteckt bzw. angeschraubt ist, ist in einen Spritzenprotektor eingesetzt, welcher im wesentlichen aus Aufnahme 4 und Einsatz 5 besteht. Der Einsatz 5, welcher den Spritzenkörper 1 hält, ist in der Aufnahme 4 nach Art zweier ineinander gesteckter Hohlkörper geführt. Eine vorgespannte Feder 6 hält den Einsatz 5 in einer durch einen Anschlag 7 vorgegebenen Endposition (nachfolgend "geschützte Position"), so daß sich die aus dem Einsatz 5 hervorragende Kanüle 2 in voller Länge innerhalb der Aufnahme 4 befindet. Vor Anwendung der Spritze wird der Einsatz 5 gegen die Federkraft der Feder 6 nach unten gedrückt, und der Rand 8 des Einsatzes 5 rastet in die Halterungen 9 ein. Daneben existieren auch Versionen ohne Einrastungen. In dieser Position (nachfolgend "Injektionsposition") ragt die Kanüle 2 aus der Aufnahme 4 hervor, und die Injektion kann vorgenommen werden. Ist die Kanüle 2 vor Anwendung mit einer festen Schutzkappe geschützt, was in der Praxis häufig der Fall ist, so kann auch die Anordnung von vornherein in Injektionsposition sein. In jedem Falle bietet der Spritzenprotektor Schutz vor Verletzung nach Anwendung der Spritze, wenn der Einsatz 5 aus den Halterungen 9 ausrastet undvon der Feder 6 in die geschützte Position zurückgeführt wird.

Das Problem bei der Verwendung mit selbstüberlappenden, Belegabschnitte enthaltenden Etiketten ausgestatteter Spritzenkörper besteht nun darin, daß die Belegabschnitte nicht entnommen werden können, solange dieser in den Spritzenprotektor eingesetzt ist. Die vorherige Entnahme des Spritzenkörpers aus dem Spritzenprotektor ist dagegen nicht vorgesehen, wäre zudem umständlich und bürge die Gefahr der Verletzung an der Kanülenspitze, insbesondere bei herrschender Eile.

Es ist daher Aufgabe der vorliegenden Erfindung, eine einen Spritzenprotektor umfassende Spritzenanordnung zu schaffen, welche die Entnahme von mindestens einem auf dem Spritzenkörper aufetikettierten Belegabschnitt erlaubt, ohne den Spritzenkörper aus dem Spritzenprotektor herausnehmen zu müssen. Die Handhabung soll dabei möglichst sicher und komfortabel sein.

Gemäß einem Aspekt der Erfindung wird die Aufgabe durch eine Spritzenanordnung nach Anspruch 1 gelöst.

Ist das Etikett bestimmungsgemäß selbstüberlappend auf den Spritzenkörper aufgeklebt, so befindet sich die nichtklebende Anfaßlasche am freien Ende. Die Anfaßlasche muß ausreichend Fläche aufweisen, damit sie durch eine seitliche Öffnung des Spritzenprotektors ohne Ablösen des Etiketts herausragen und leicht gegriffen werden kann. Hierfür ist es vorteilhaft, wenn die größte Ausdehnung der Anfaßlasche in Streifenlängsrichtung mindestens 1 mm, besonders vorteilhaft mindestens 2,5 mm beträgt.

Gegebenenfalls kann die Anfaßlasche nach Einsetzen des Spritzenkörpers in den Spritzenprotektor durch die seitliche Öffnung desselben durch leichte Drehung des Spritzenkörpers relativ zum Spritzenprotektor gewissermaßen herausgeschält und dann ergriffen werden, da die Anfaßlasche nach Aufetikettieren auf einen Spritzenkörper etwas absteht. Insbesondere wenn die Anfaßlasche mit einer geeigneten Schrägung oder Rundung versehen ist, läuft das Etikett auf für den Fachmann völlig überraschende Weise beim Einführen des etikettierten Spritzenkörpers in den engen Spritzenprotektor dennoch nicht Gefahr, von seinem freien Ende her verknickt oder sonstwie beschädigt zu werden, da sich die Anfaßlasche insbesondere aufgrund ihrer bevorzugten Gestaltung während des Einführvorgangs an den etikettierten Spritzenkörper anlegt. Hierzu ist das Etikett vorzugsweise so aufzukleben, daß die Ausdehnung der Anfaßlasche in Streifenlängsrichtung von der in Einführrichtung vorne liegenden Etikettenkante her kontinuierlich zunimmt, und zwar idealerweise über den Großteil der Breite des Etikettenstreifens. Am geringsten ist die Gefahr einer Beschädigung des Etiketts von seinem freien Ende her, wenn die Ausdehnung der Anfaßlasche in Streifenlängsrichtung an der in Einführrichtung vorne liegenden Etikettenkante weniger als 10 Prozent Ihrer größten Ausdehnung in Streifenlängsrichtung beträgt. Idealerweise erweitert sich die Anfaßlasche in über die Streifenbreite langsam, ausgehend von einer verschwindenden Ausdehnung in Streifenlängsrichtung.

Ist der etikettierte Spritzenkörper in den Spritzenprotektor eingesetzt, kann das Etikett durch Anziehen an der Anfaßlasche von dem Spritzenkörper teilweise abgerollt werden. Dabei wird das Etikett durch eine Öffnung im Einsatz des Spritzenprotektors (in der geschützten Position) bzw. durch Öffnungen in Einsatz und Aufnahme des Spritzenprotektors (in Injektionsposition) so weit herausgezogen, bis der Belegabschnitt bzw. die Belegabschnitte entlang der Trennlinien abreißbar sind. Somit ist es nicht erforderlich, den Spritzenkörper aus dem Spritzenprotektor herauszunehmen, um an die Belegabschnitte zu gelangen. Letztere lassen sich somit sicher, einfach, schnell, komfortabel und mit wenig Aufwand entnehmen.

Nachfolgend werden erfindungsgemäße Ausführungsbeispiele anhand der zugehörigen Zeichnungen ausführlicher beschrieben. Die Zeichnungen sind nicht maßstabsgerecht und rein schematisch aufzufassen. Insbesondere sind die Schichtdicken der verschiedenen Etikettenlagen aus Anschaulichkeitsgründen stark übertrieben dargestellt. Bezugszeichen einander entsprechender Merkmale werden für alle Figuren durchgehend beibehalten.
Fig. 1 zeigt eine perspektivische Ansicht eines erfindungsgemäßen Etiketts mit zwei Belegabschnitten, welches auf einem Stück Abziehmaterial angeordnet ist, wobei das Längenzu-Breiten-Verhältnis des Etikettenstreifens verzerrt dargestellt ist (verkürzte Streifenlänge);
Fig. 2a-c zeigen stark vergrößert jeweils den oberen Bereich eines erfindungsgemäß etikettierten Spritzenkörpers sowie des Einsatzes und der Aufnahme einer erfindungsgemäßen Spritzenanordnung;
Fig. 3 zeigt das prizipielle Zusammenwirken eines Spritzenprotektors mit einer Spritze gemäß dem Stand der Technik, welches gleichermaßen für die erfindungsgemäße Spritzenanordnung zutrifft;
Fig. 4 zeigt eine Schnittansicht durch den Spritzenkörper aus Fig. 2a in der durch die Linie A-A' angedeuteten, zur Längsachse des Spritzenkörpers senkrechten Schnittebene, sowie eine durch zwei verbundene Rechtecke angedeutete Ausschnittsvergrößerung;
Fig. 5 zeigt die in Fig. 2a-c dargestellten Teile der Spritzenanordnung ineinander eingesetzt, wobei die Lasche bereits etwas aus der seitlichen Öffnung der Aufnahme herausragt (Transparenz der Teile der Spritzenanordnung in der Darstellung nicht berücksichtigt);
Fig. 6 zeigt einen Auschnitt der Spritzenanordnung aus Fig. 5, wobei das Etikett weiter herausgezogen ist, so daß beide Belegabschnitte sichtbar sind;
Fig. 7 zeigt ein Etikett, welches sich von dem in Fig. 1 dargestellten Etikett durch eine alternative Anordnung der Belegabschnitte unterscheidet;
Fig. 8a-e zeigen sechs verschiedene Varianten der Gestaltung der Anfaßlasche eines erfindungsgemäßen Etiketts;
Fig. 9 zeigt eine perspektivische Ansicht eines weiteren erfindungsgemäßen Etiketts mit zwei Belegabschnitten, wobei das Längen-zu-Breiten-Verhältnis des Etikettenstreifens verzerrt dargestellt ist (verkürzte Streifenlänge);
Fig. 10 zeigt ähnlich Fig. 4 eine Schnittansicht durch einen etikettierten Spritzenkörper aus in zur Längsachse des Spritzenkörpers senkrechten Schnittebene, sowie eine durch zwei verbundene Rechtecke angedeutete Ausschnittsvergrößerung, wobei das Etikett ähnlich der Ausführungsform aus Fig. 9 gestaltet ist.

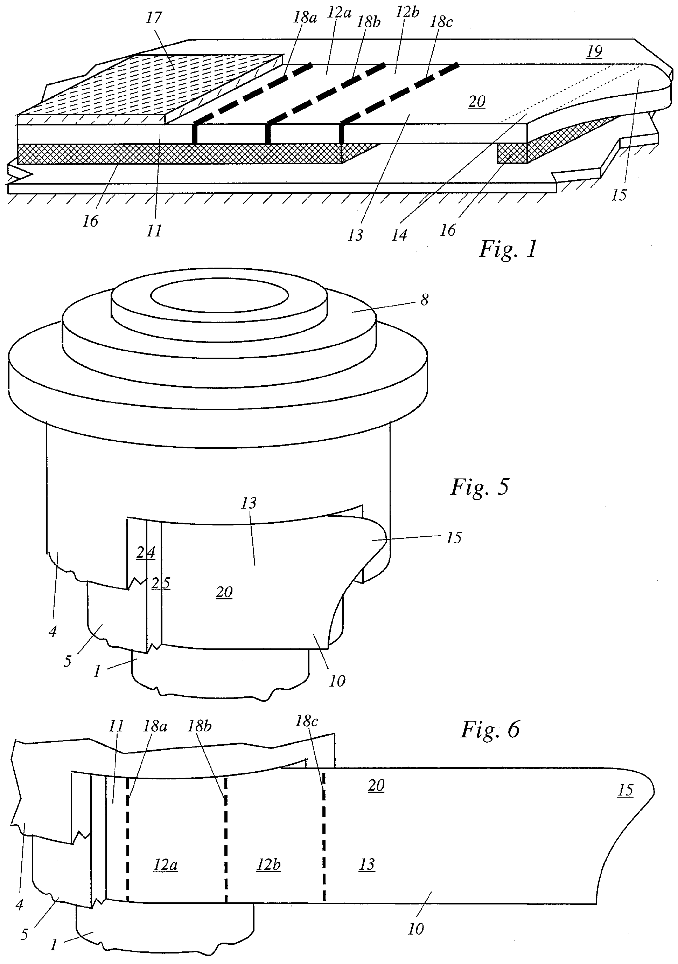

Das in Fig. 1 dargestellte Etikett ist streifenförmig ausgeführt. In Streifenlängsrichtung (von links nach rechts bei der gewählten Darstellung) aufeinanderfolgend weist es einen Grundabschnitt 11, zwei Belegabschnitte 12a, 12b, einen Schutzabschnitt 13, einen Halteabschnitt 14 und eine Anfaßlasche 15 auf. Die genannten Bereiche 11, 12a, 12b, 13, 14, 15 sind vorteilhafterweise im wesentlichen aus einer durchgehenden Kunststoffolienlage 20 gebildet, welche transparent oder opak sein kann. Ebenso ist eine Herstellung des Etiketts auf Papierbasis oder als mehrlagiger Folienverbund möglich. Die den Halteabschnitt 14 vom Schutzabschnitt 13 bzw. der Anfaßlasche 15 trennenden feinpunktierten Linien dienen der Anschaulichkeit und stellen kein körperliches Merkmal der Erfindung dar.

Die Darstellung ist aus Anschaulichkeitsgründen so verzerrt, daß die Streifenbreite im Verhältnis zur Streifenlänge übertrieben, die Dicke der einzelnen Lagen im Verhältnis zu den übrigen Dimensionen stark übertrieben abgebildet ist.

Unterseitig ist das Etikett mit einer Haftklebstoffschicht 16 versehen, welche im Bereich des Schutzabschnitts 13 und der Anfaßlasche 15 jeweils unterbrochen ist. Alternativ kann die gesamte Etikettenunterseite klebstoffbeschichtet sein, wobei die Klebstoffbeschichtung im Bereich des Schutzabschnitts 13 und der Anfaßlasche 15 mittels eines sogenannten "Klebstoffkillers" neutralisiert, d.h. in einen nichtadhäsiven Zustand übergeführt ist.

Der Grundabschnitt 11 ist oberseitig mit eine klebstoffabweisenden Beschichtung 17 (beispielsweise einer Silikonschicht) versehen, welche mindestens die gleiche Flächenausdehnung besitzt, wie die Gesamtheit der Belegabschnitte 12a, 12b. In manchen Fällen kann es aber auch vorteilhaft sein, wenn die Ausdehnung der klebstoffabweisenden Beschichtung etwas kleiner ist, so daß kleine Bereiche (gegenüber dem silikonisierten Bereich) erhöhter Haftung für guten Zusammenhalt vor dem Ablösen der Beelegabschnitte sorgen.

Die Belegabschnitte 12a, 12b sind entlang der Trennlinien 18a, 18b, 18c, welche sich in Streifenquerrichtung über die gesamte Breite des Etiketts erstrecken, vom Grundabschnitt 11 bzw. voneinander bzw. vom Schutzabschnitt 13 trennbar. Die Trennlinien 18a, 18b, 18c können als Perforationen, Stanzungen oder andere den Abriß erleichternde lokale Schwächungen der Folienlage 20 ausgeführt sein.

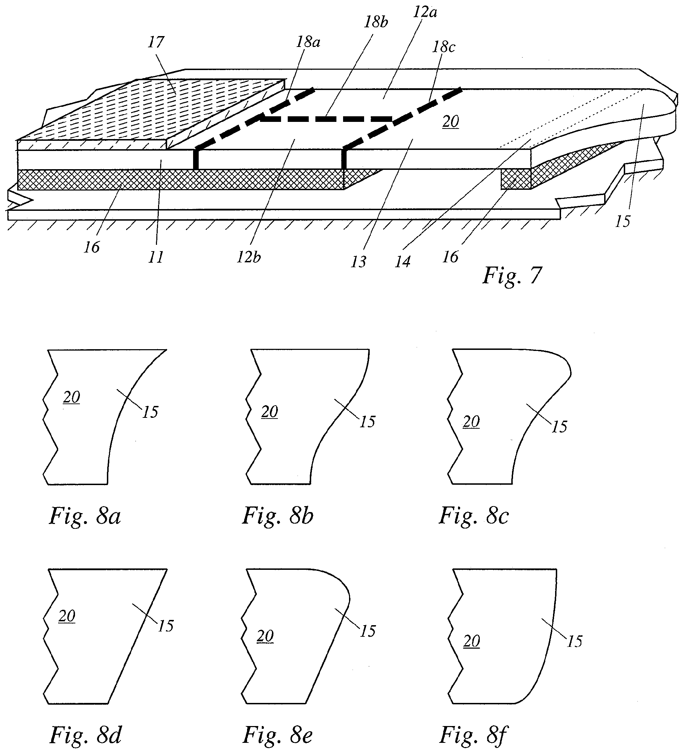

Der Schutzabschnitt 13 ist in Streifenlängsrichtung breiter als die Gesamtheit der Belegabschnitte 12a, 12b. Der Halteabschnitt 14 ist in Streifenlängsrichtung schmäler als die restlichen Abschnitte 11, 12a, 12b, 13. Die Anfaßlasche 15 verbreitert sich in Streifenlängsrichtung kontinuierlich über den Großteil der Breite des Etikettenstreifens. Diese flügelartige Gestalt der Anfaßlasche 15 sorgt nach erfindungsgemäßer Etikettierung eines Spritzenkörpers 1 dafür, daß sie sich beim Einführen des Spritzenkörpers 1 in einen engen Spritzenprotektor an den etikettierten Spritzenkörper anlegt, wie unten näher erläutert wird. Entscheidend dabei ist, daß das Etikett so angebracht wird, daß der in Fig. 1 vordere Etikettenrand, d.h. der in Streifenlängsrichtung verlaufende Rand des Etiketts 10, von welchem ausgehend sich die Anfaßlasche 15 kontinuierlich über die Mitte (in Streifenquerrichtung) des Etiketts hinaus verbreitert, beim Einführen des Spritzenkörpers 1 in den Spritzenprotektor der vorauseilende Rand ist. Im Zusammenhang mit der vorliegenden Erfindung wird eine kontinuierliche Verbreiterung vom Rand her über die Mitte (in Streifenquerrichtung) des Etiketts hinaus als gleichbedeutend mit einer Verbreiterung über den Großteil der Breite des Etikettenstreifens angesehen. Verschiedene alternative Formgebungen der Anfaßlasche 15, welche dieses Kriterium erfüllen, sind in Fig. 8a-8e dargestellt.

Üblicherweise ist das Etikett mit Text- und/oder Bildinformation (nicht dargestellt) bedruckt. Im Bereich des Grundabschnitts 11 ist ein derartiger Aufdruck aus drucktechnischen Gründen normalerweise unterhalb der klebstoffabweisenden Beschichtung 17 ausgeführt. Die Information auf dem ersten Belegabschnitt 12a ist üblicherweise im wesentlichen identisch mit der Information auf dem zweiten Belegabschnitt 12b und findet eine Entsprechung in der Information auf dem Grundabschnitt 11, um eine (auch nachträgliche) Zuordnung der Belegabschnitte 12a, 12b zueinander und zum etikettierten Spritzenkörper 1 zu ermöglichen. Die Information auf dem Schutzabschnitt 13 wird meist der Information auf dem Grundabschnitt 11 entsprechen, da nach Etikettieren des Spritzenkörpers 1 zunächst nur ersterer, nicht jedoch letzterer zu sehen ist, wie unten erläutert wird. Die aufgedruckte Information kann unter anderem Wirkstoff-, Mengen- und Konzentrationsangaben, Haltbarkeitsdaten, Chargennummern, Barcodes, Handels- und Erzeuger-Marken usw. umfassen. Ferner können auch flächige Drucke als Farbhintergrund vorgesehen sein, insbesondere dann, wenn die Kunststoffolienlage 20 transparent ausgeführt ist.

Über die Haftklebstoffschicht 16 ist das Etikett auf einem ausschnittsweise dargestellten Stück Abziehmaterial 19 angeordnet, von welchem es leicht abgelöst werden kann, um bestimmungsgemäß auf einen Spritzenkörper 1 aufgeklebt zu werden.

Fig. 2a zeigt einen Spritzenkörper 1, auf welchen ein Etikett aufgeklebt ist, welches im wesentlichen wie das in Fig. 1 dargestellte Etikett ausgebildet ist. Aus Anschaulichkeitsgründen ist der Spritzenkörper 1, wie auch der in Fig. 2b abgebildete Einsatz 5 und die in Fig. 2a abgebildete Aufnahme, stark vergrößert dargestellt, und zwar auch im Verhältnis zur Größe des Etiketts 10, welches in der tatsächlichen Anwendung üblicherweise den Großteil der Mantelfläche 21 des Spritzenkörpers 1 bedeckt.

Eine zur Längsachse des Spritzenkörpers 1 senkrechte Schnittebene ist durch die strichpunktierte Linie A-A' angedeutet. Eine entsprechende Schnittansicht (in Pfeilrichtung) ist in Fig. 4 dargestellt mit im Verhältnis zu Fig. 2a stark übertreibenen Schichtdicken der Lagen 16, 17, 20, 26 des Etiketts 10. Eine weitere Ausschnittsvergrößerung ist durch die zwei fett umrandeten Rechtecke im oberen Abschnitt der Fig. 4 illustriert, wobei das obere der beiden Rechtecke in Vergrößerung den gleichen Auschnitt zeigt, wie das untere.

Das Etikett überlappt sich zweimal selbst. Dabei liegt die Haftklebstoffschicht 16 im Bereich der Belegabschnitte 12a, 12b auf der klebstoffabweisenden Beschichtung 17 des Grundabschnitts 11 auf, welcher seinerseits auf die Mantelfläche 21 des Spritzenkörpers 1 aufgeklebt ist. Der Schutzabschnitt 13 überdeckt die Belegabschnitte 12a, 12b. Im Unterschied zu Fig. 1 ist die nichtklebende Unterseite des Schutzabschnitts 13 und der Anfaßlasche 15 nicht durch eine Unterbrechung der Haftklebstoffschicht 16 realisiert, sondern dadurch, daß die Haftklebstoffschicht 16 in den entsprechenden Bereichen 26a, 26b durch Einsatz eines sogenannten "Klebstoffkillers" neutralisiert, d.h. in einen nichtadhesiven Zustand übergeführt ist.

Der Halteabschnitt 14 klebt über einen schmalen nicht-neutralisierten Bereich der Haftklebstoffschicht 16 auf einem an den Belegabschnitt 12b angrenzenden Bereich des Schutzabschnitts 13, um die überlappende Anordnung des Etiketts zusammenzuhalten und gegen unbeabsichtigtes Abrollen zu sichern.

Sowohl der Spritzenkörper 1 in Fig. 2a, als auch der Einsatz 5 in Fig. 2b und die Aufnahme 4 in Fig. 2c, welche Teile des Spritzenprotektors der erfindungsgemäßen Spritzenanordnung darstellen, sind nur ausschnittsweise, d.h. nach unten zu abgebrochen dargestellt. Der Einsatz 5 ist in der Aufnahme 4 geführt, wie dies in Fig. 3 dargestellt ist. Auch ansonsten ist das Grundprinzip des Zusammenwirkens zwischen Spritzenkörper 1, Einsatz 5 und Aufnahme 4 der erfindungsgemäßen Spritzen-anordnung grundsätzlich gleich wie im Stand der Technik und oben anhand Fig. 3 beschrieben. Aus dem Stand der Technik bereits bekannte mechanische Details wie der Anschlag 7 und die Halterungen 9 sind in Fig. 2b und Fig. 2c der Einfachheit halber nicht dargestellt.

Aufnahme 4 und Einsatz 5 bestehen vorzugsweise aus transparentem Kunststoff, so daß der Aufdruck (nicht dargestellt) auf dem Schutzabschnitt 13 lesbar bleibt, wenn der etikettierte Spritzenkörper 1 in den Einsatz 5 eingeführt ist. Die Öffnungen 24, 25 dienen dazu, den Zugriff auf die Anfaßlasche 15 auch dann zu ermöglichen, wenn der etikettierte Spritzenkörper 1 in den Einsatz 5 eingeschoben ist, und sich der im wesentlichen aus Aufnahme 4 und Einsatz 5 bestehende Spritzenprotektor in Injektionsposition befindet. Entsprechend ist der Zugriff auf die Anfaßlasche 15 durch die Öffnung 25 des Einsatzes 5 alleine möglich, wenn sich der Spritzenprotektor in geschützter Position befindet.

Der etikettierte Spritzenkörper 1 wird von oben in den Einsatz 5 eingeschoben. Dabei legt sich die Anfaßlasche 15, welche aufgrund ihrer nichtklebenden Unterseite normalerweise leicht absteht, wie in Fig. 4 angedeutet, aufgrund ihrer erfindungsgemäßen Formgebung an den Schutzabschnitt 13 an. Somit wird verhindert, daß beim Einschieben des etikettierten Spritzenkörpers 1 in den Einsatz 5 der Haltebereich 14 ablöst, und das Etikett verknittert, wie es bei ungünstigerer Gestaltung des freien Etikettenendes der Fall wäre.

Fig. 5 und Fig. 6 zeigen ausschnittsweise die erfindungsgemäße Spritzenanordnung mit bereits eingeschobenem Spritzenkörper 1 in Injektionsposition. Auf grafische Andeutung der Transparenz der Bestandteile wurde der Übersichtlichkeit halber verzichtet.

In Fig. 5 ragt die Anfaßlasche 15 durch die Öffnungen 24, 25 hindurch bereits etwas aus dem aus Einsatz 5 und Aufnahme 4 gebildeten Spritzenprotektor heraus, so daß sie sich leicht ergreifen läßt. Dieser Zustand kann bei der dargestellten Wicklung des Etiketts gegen den Uhrzeigersinn leicht herbeigeführt werden, indem man den etikettierten Spritzenkörper 1 relativ zu Einsatz 5 und Aufnahme 4 etwas gegen den Uhrzeigersinn dreht.

Fig. 6 zeigt den Zustand nach Anziehen an der Anfaßlasche 15. Das Etikett ist nun weiter abgewickelt und weiter aus dem aus Einsatz 5 und Aufnahme 4 gebildeten Spritzenprotektor herausgezogen, so daß der Belegabschnitt 12b bereits frei zugänglich ist. Durch weiteres Anziehen an der Anfaßlasche 15 wird auch der andere Belegabschnitte 12a frei zugänglich.

Fig. 7 zeigt eine alternative Anordnung der Belegabschnitte 12a, 12b. Die Darstellung entspricht im wesentlichen Fig. 1. Die Trennlinien 18a, 18c, welche über die gesammte Breite des Etiketts in Streifenquerrichtung verlaufen, ermöglichen die Trennung der Belegabschnitte 12a, 12b vom Grundabschnitt 11 respektive Schutzabschnitt 13. Voneinander sind die Belegabschnitte 12a, 12b über die in Streifenlängsrichtung verlaufende Trennlinie 18b abtrennbar. Darüber hinaus sind auch andere Anordnungen eines, zweier oder mehrerer Belegabschnitte möglich.

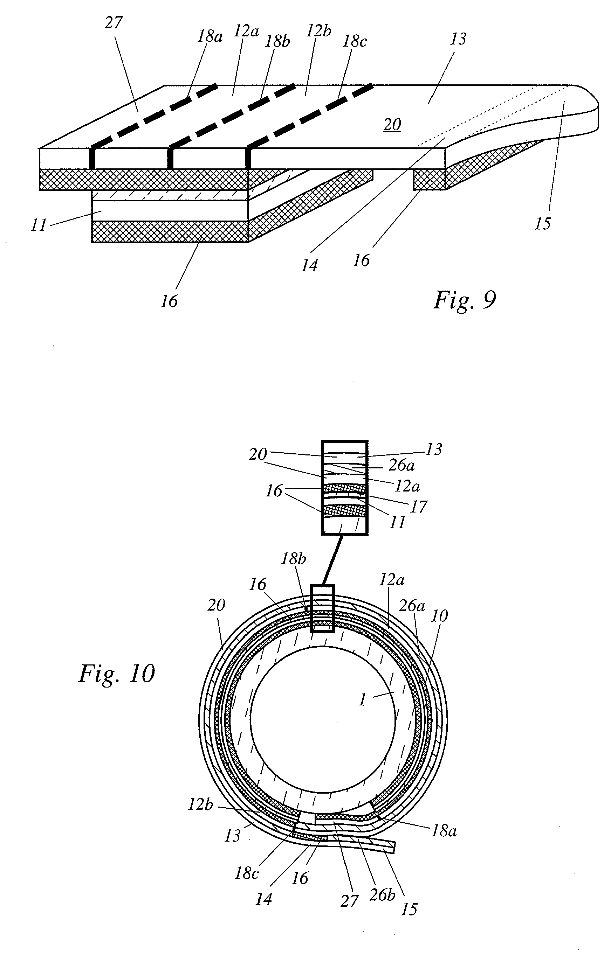

Wie das in Fig. 1 dargestellte Etikett, ist auch das in Fig. 9 dargestellte Etikett streifenförmig ausgeführt. Die Darstellung ist aus Anschaulichkeitsgründen so verzerrt, daß die Streifenbreite im Verhältnis zur Streifenlänge übertrieben, die Dicke der einzelnen Lagen im Verhältnis zu den übrigen Dimensionen stark übertrieben abgebildet ist.

In Streifenlängsrichtung (von links nach rechts bei der gewählten Darstellung) aufeinanderfolgend weist das Etikett einen Anfangsabschnitt 27, zwei Belegabschnitte 12a, 12b, einen Schutzabschnitt 13, einen Halteabschnitt 14 und eine Anfaßlasche 15 auf. Die genannten Bereiche 27, 12a, 12b, 13, 14, 15 sind vorteilhafterweise im wesentlichen aus einer durchgehenden Kunststoffolienlage 20 gebildet, welche transparent oder opak sein kann. Ebenso ist eine Herstellung des Etiketts auf Papierbasis oder als mehrlagiger Folienverbund möglich. Die den Halteabschnitt 14 vom Schutzabschnitt 13 bzw. der Anfaßlasche 15 trennenden feinpunktierten Linien dienen der Anschaulichkeit und stellen kein körperliches Merkmal der Erfindung dar.

Das in Fig. 9 dargestellte Etikett unterscheidet sich von der in Fig. 1 dargestellten Ausführungsform dadurch, daß der Grundabschnitt 11 unter und nicht neben den Belegabschnitten 12a, 12b angeordnet ist. Im Gegensatz zu dem Etikett aus Fig. 1, welches aus einem einzigen Folienzuschnitt herstellbar ist, werden bei dem in Fig. 9 dargestellten Etikett Grundabschnitt 11 und die Gesamtheit der restlichen Abschnitte 12a, 12b, 13, 14, 15 aus jeweils einem eigenen Folienzuschnitt hergestellt. Der unterseitig klebstoffbeschichtete, die Bereiche 12a, 12b, 13, 14, 15 bildende Folienzuschnitt wird auf den oberseitig klebstoffabweisend beschichteten Grundabschnitt 11 aufgespendet, welcher auf einem Stück Abziehmaterial angeordnet ist, welches im Gegensatz zu Fig. 1 in Fig. 9 nicht dargestellt ist.

Unterseitig ist jeder Folienzuschnitt des Etiketts mit einer Haftklebstoffschicht 16 versehen, welche im Bereich des Schutzabschnitts 13 und der Anfaßlasche 15 jeweils unterbrochen ist. Alternativ kann die gesamte Etikettenunterseite klebstoffbeschichtet sein, wobei die Klebstoffbeschichtung im Bereich des Schutzabschnitts 13 und der Anfaßlasche 15 mittels eines sogenannten "Klebstoffkillers" neutralisiert, d.h. in einen nichtadhäsiven Zustand übergeführt ist.

Der Grundabschnitt 11 ist bei vorliegendem Ausführungsbeispiel oberseitig mit einer klebstoffabweisenden Beschichtung 17 (beispielsweise einer Silikonschicht) versehen, welche mindestens die gleiche Flächenausdehnung besitzt, wie die Gesamtheit der Belegabschnitte 12a, 12b. In anderen Fällen kann es aber auch vorteilhaft sein, wenn die Ausdehnung der klebstoffabweisenden Beschichtung etwas kleiner ist, so daß kleine Bereiche (gegenüber dem silikonisierten Bereich) erhöhter Haftung für guten Zusammenhalt vor dem Ablösen der Belegabschnitte sorgen.

Die Belegabschnitte 12a, 12b sind entlang der Trennlinien 18a, 18b, 18c, welche sich in Streifenquerrichtung über die gesamte Breite des Etiketts erstrecken, vom Anfangsabschnitt 11 bzw. voneinander bzw. vom Schutzabschnitt 13 trennbar. Die Trennlinien 18a, 18b, 18c können als Perforationen, Stanzungen oder andere den Abriß erleichternde lokale Schwächungen der Folienlage 20 ausgeführt sein.

Der Schutzabschnitt 13 ist in Streifenlängsrichtung breiter als die Gesamtheit der Belegabschnitte 12a, 12b. Der Halteabschnitt 14 ist in Streifenlängsrichtung schmäler als die Abschnitte 11, 12a, 12b, 13. Die Anfaßlasche 15 verbreitert sich in Streifenlängsrichtung kontinuierlich über den Großteil der Breite des Etikettenstreifens. Diese flügelartige Gestalt der Anfaßlasche 15 sorgt nach erfindungsgemäßer Etikettierung eines Spritzenkörpers 1 dafür, daß sie sich beim Einführen des Spritzenkörpers 1 in einen engen Spritzenprotektor an den etikettierten Spritzenkörper anlegt, wie bereits oben näher erläutert ist. Entscheidend dabei ist, daß das Etikett so angebracht wird, daß der in Fig. 1 vordere Etikettenrand, d.h. der in Streifenlängsrichtung verlaufende Rand des Etiketts 10, von welchem ausgehend sich die Anfaßlasche 15 kontinuierlich über die Mitte (in Streifenquerrichtung) des Etiketts hinaus verbreitert, beim Einführen des Spritzenkörpers 1 in den Spritzenprotektor der vorauseilende Rand ist. Auch hier sind die verschiedenen alternative Formgebungen der Anfaßlasche 15 gemäß Fig. 8a-8e möglich.

Üblicherweise ist das Etikett mit Text- und/oder Bildinformation (nicht dargestellt) bedruckt. Im Bereich des Grundabschnitts 11 ist ein derartiger Aufdruck aus drucktechnischen Gründen normalerweise unterhalb der klebstoffabweisenden Beschichtung 17 ausgeführt. Die Information auf dem ersten Belegabschnitt 12a ist üblicherweise im wesentlichen identisch mit der Information auf dem zweiten Belegabschnitt 12b und findet eine Entsprechung in der Information auf dem Grundabschnitt 11, um eine (auch nachträgliche) Zuordnung der Belegabschnitte 12a, 12b zueinander und zum etikettierten Spritzenkörper 1 zu ermöglichen. Die Information auf dem Schutzabschnitt 13 wird meist der Information auf dem Grundabschnitt 11 entsprechen, da nach Etikettieren des Spritzenkörpers 1 zunächst nur ersterer, nicht jedoch letzterer zu sehen ist. Die aufgedruckte Information kann unter anderem Wirkstoff-, Mengen- und Konzentrationsangaben, Haltbarkeitsdaten, Chargennummern, Barcodes, Handels- und Erzeuger-Marken usw. umfassen. Ferner können auch flächige Drucke als Farbhintergrund vorgesehen sein, insbesondere dann, wenn die Kunststoffolienlage 20 transparent ausgeführt ist.

Der Anfangsabschnitt 27 ist in der Regel deutlich kürzer als der Grundabschnitt 11 ausgeführt. Vorzugsweise ist er kürzer als ein Fünftel des Grundabschnitts 11 bzw. kürzer als ein Zehntel des Umfangs des zu etikettierenden Bereichs des bestimmungsgemäßen Spritzenkörpers 1. Der Anfangsabschnitt 27 verbessert die Verspendbarkeit des Etiketts, kann jedoch oft auch entbehrlich sein.

Gegenüber der in Fig. 1 dargestellten Ausführungsform besitzt das Etikett aus Fig. 9 den Vorteil, daß der Etikettenstreifen insgesamt kürzer ist, wodurch das Etikett insbesondere bei maschineller Verspendung einfacher und schneller auf den Spritzenkörper 1 appliziert werden kann. Ferner besteht die Möglichkeit, Grundabschnitt 11 und die übrigen Abschnitte 27, 12a, 12b, 13, 14, 15 aus unterschiedlichen Folienmaterialien zu fertigen, wodurch, neben anderen denkbaren Vorteilen, unter Umständen die Gesamtdicke der bestimmungsgemäß auf den Spritzkörper 1 aufgewickelten Etikettenlagen etwas dünner ausfallen kann. Dagegen ist der Aufwand zur Herstellung des Etiketts aus Fig. 1 geringer als zur Herstellung des Etiketts aus Fig. 9.

Fig. 10 zeigt analog Fig. 4 eine Schnittansicht eines etikettierten Spritzenkörpers 1. Das selbstüberlappend darauf angebrachte Etikett ist im wesentlichen ausgeführt wie das in Fig. 9 dargestellte Etikett. Eine Ausschnittsvergrößerung ist wie in Fig. 4 durch die zwei fett umrandeten Rechtecke im oberen Abschnitt der Fig. 10 illustriert, wobei das obere der beiden Rechtecke in Vergrößerung den gleichen Auschnitt zeigt, wie das untere.

Die Haftklebstoffschicht 16 im Bereich der Belegabschnitte 12a, 12b liegt auf der klebstoffabweisenden Beschichtung 17 des Grundabschnitts 11 auf, welcher seinerseits auf die Mantelfläche 21 des Spritzenkörpers 1 aufgeklebt ist. Der Schutzabschnitt 13 überdeckt aufgrund überlappender Anbringung die Belegabschnitte 12a, 12b. Im Unterschied zu Fig. 9 ist die nichtklebende Unterseite des Schutzabschnitts 13 und der Anfaßlasche 15 nicht durch eine Unterbrechung der Haftklebstoffschicht 16 realisiert, sondern dadurch, daß die Haftklebstoffschicht 16 in den entsprechenden Bereichen 26a, 26b durch Einsatz eines sogenannten "Klebstoffkillers" neutralisiert, d.h. in einen nichtadhesiven Zustand übergeführt ist.

Der Halteabschnitt 14 klebt über einen schmalen nicht-neutralisierten Bereich der Haftklebstoffschicht 16 auf einem an den Belegabschnitt 12b angrenzenden Bereich des Schutzabschnitts 13, um die überlappende Anordnung des Etiketts zusammenzuhalten und gegen unbeabsichtigtes Abrollen zu sichern.

Zur besseren Fixierung der Belegabschnitte 12a, 12b sowie zur Erleichterung des Etikettierens insbesondere bei maschineller Verspendung haftet der Anfangsabschnitt 27 direkt auf dem Spritzenkörper 1.

Anhand eines Vergleichs von Fig. 4 und Fig. 10 erkennt man, daß bei bestimmungsgemäßer Anbringung der Etiketten sich die beiden Anordnungen der Etikettenlagen auf dem etikettierten Spritzenkörper 1 trotz unterschiedlich ausgeführter Etiketten (vgl. Figuren 1 und 9) kaum voneinander unterscheiden. Beide Spritzenkörper 1 sind gleichermaßen in den Einsatz 5 eines Spritzenprotektors einschiebbar und bieten im wesentlichen die gleiche Funktionalität.

## Patentansprüche

1. Spritzenanordnung, aufweisend:
- einen als Spritzenprotektor ausgebildeten Außenkörper mit einer mindestens eine seitliche Öffnung (24) aufweisenden Aufnahme (4) und einem mindestens eine seitliche Öffnung (25) aufweisendenden Einsatz (5),
- einen als Spritzenkörper (1) ausgebildeten Innenkörper, der in den Spritzenprotektor eingesetzt ist, wobei der Spritzenkörper (1) mit einem Etikett versehen ist,
wobei das Etikett in Streifenlängsrichtung nebeneinander folgende Abschnitte aufweist:
- mindestens einen Belegabschnitt (12a, 12b), welcher in Streifenlängsrichtung entlang mindestens einer in Streifenquerrichtung verlaufenden Trennlinie (18a, 18b, 18c) vom restlichen Etikett abtrennbar ist und unterseitig zumindest teilfächig eine Klebstoffbeschichtung (16) aufweist,
- eine Anfaßlasche (15), welche unterseitig nichtklebend ausgeführt ist, und welche von dem mindestens einen Belegabschnitt (12a, 12b) aus gesehen in Streifenlängsrichtung endständig am Etikett angeordnet ist,
- einen Grundabschnitt (11), welcher oberseitig zumindest teilflächig eine klebstoffabweisende Oberfläche und unterseitig zumindest teilfächig eine Klebstoffbeschichtung (16) aufweist,
wobei das Etikett durch Anziehen an der Anfaßlasche (15) durch die seitlichen Öffnungen (24, 25) des Einsatzes (5) bzw. der Aufnahme (4) hindurch teilweise von dem Spritzenkörper (1) abrollbar und aus dem Spritzenprotektor ausziehbar ist, so daß die Belegabschnitte (12a, 12b) abgetrennt werden können, ohne den Spritzenkörper (1) aus dem Spritzenprotektor entnehmen zu müssen.

2. Spritzenanordnung gemäß Anspruch 1, wobei der Grundabschnitt (11) in Streifenlängsrichtung neben dem Belegabschnitt bzw. den Belegabschnitten (12a, 12b) angeordnet ist, der Belegabschnitt bzw. die Belegabschnitte (12a, 12b) zweiseitig entlang in Streifenquerrichtung verlaufender Trennlinien (18a, 18b, 18c) vom restlichen Etikett abtrennbar sind, und die Anfaßlasche (15) in Streifenlängsrichtung auf der dem Grundabschnitt (11) gegenüberliegenden Seite des Etiketts angeordnet ist.

3. Spritzenanordnung gemäß Anspruch 1, wobei der Grundabschnitt (11) unter dem Belegabschnitt bzw. den Belegabschnitten (12a, 12b) angeordnet ist, und die Klebstoffbeschichtung (16) des Belegabschnitts bzw. der Belegabschnitte (12a, 12b) auf der klebstoffabweisenden Oberfläche des Grundabschnitts (11) haften.

4. Spritzenanordnung gemäß Anspruch 3, welches einen Anfangsabschnitt (26) aufweist, welcher in Streifenlängsrichtung auf der der Anfaßlasche (15) gegenüberliegenden Seite des Etiketts endständig angeordnet ist.

5. Spritzenanordnung gemäß einem der vorangehenden Ansprüche, wobei das Etikett ferner aufweist
- einen Schutzabschnitt (13), welcher an einer Seite an einen Belegabschnitt (12b) angrenzt und unterseitig zumindest zum überwiegenden Teil seiner Fläche nichtklebend ausgeführt ist,
- einen Halteabschnitt (14), welcher sich in Streifenlängsrichtung an der dem Belegabschnitt bzw. den Belegabschnitten (12a, 12b) gegenüberliegenden Seite an den Schutzabschnitt (13) anschließt und unterseitig zumindest teilfächig eine Klebstoffbeschichtung (16) aufweist,
wobei sich die Anfaßlasche (15) in Streifenlängsrichtung an der dem Schutzabschnitt (13) gegenüberliegenden Seite an den Halteabschnitt (14) anschließt.

6. Spritzenanordnung gemäß Anspruch 5, wobei die klebstoffabweisende Oberfläche und der Schutzabschnitt (13) in Streifenlängsrichtung jeweils mindestens die gleiche Ausdehnung besitzen wie der Belegabschnitt bzw. die Gesamtheit der Belegabschnitte (12a, 12b).

7. Spritzenanordnung gemäß einem der Ansprüche 5-6, wobei die Ausdehnung des Halteabschnitts (14) in Streifenlängsrichtung geringer ist als die Ausdehnung des Schutzabschnitts (13).

8. Spritzenanordnung gemäß einem einem der vorangehenden Ansprüche, wobei die Ausdehnung der Anfaßlasche (15) in Streifenlängsrichtung über einen Teil der Streifenbreite in Streifenquerrichtung kontinuierlich zunimmt.

9. Spritzenanordnung gemäß Anspruch 8, wobei die Ausdehnung der Anfaßlasche (15) in Streifenlängsrichtung über den Großteil der Streifenbreite in Streifenquerrichtung kontinuierlich zunimmt.

10. Spritzenanordnung gemäß einem der Ansprüche 8-9, wobei die geringste Ausdehnung der Anfaßlasche (15) in Streifenlängsrichtung zwischen 0 und 25 Prozent der größten Ausdehnung der Anfaßlasche (15) in Streifenlängsrichtung beträgt.

11. Spritzenanordnung gemäß Anspruch 10, wobei die geringste Ausdehnung der Anfaßlasche (15) in Streifenlängsrichtung zwischen 0 und 10 Prozent der größten Ausdehnung der Anfaßlasche (15) in Streifenlängsrichtung beträgt.

12. Spritzenanordnung gemäß einem der vorangehenden Ansprüche, wobei die größte Ausdehnung der Anfaßlasche (15) in Streifenlängsrichtung mindestens 3 Prozent der Ausdehnung des Grundabschnitts (11) in Streifenlängsrichtung beträgt.

13. Spritzenanordnung gemäß Anspruch 12, wobei die größte Ausdehnung der Anfaßlasche (15) in Streifenlängsrichtung mindestens 5 Prozent der Ausdehnung des Grundabschnitts (11) in Streifenlängsrichtung beträgt.

14. Spritzenanordnung gemäß einem der vorangehenden Ansprüche, wobei die größte Ausdehnung der Anfaßlasche (15) in Streifenlängsrichtung mindestens 1 mm beträgt.

15. Spritzenanordnung gemäß Anspruch 14, wobei die größte Ausdehnung der Anfaßlasche (15) in Streifenlängsrichtung mindestens 2,5 mm beträgt.

16. Spritzenanordnung gemäß einem der vorangehenden Ansprüche, wobei sich die Trennlinie bzw. Trennlinien (18a, 18b, 18c) in Streifenquerrichtung über das gesamte Etikett erstreckt bzw. erstrecken.

17. Spritzenanordnung gemäß einem der vorangehenden Ansprüche, wobei die klebstoffabweisende Oberfläche durch eine Silikonisierung (17) gebildet ist.

18. Spritzenanordnung gemäß einem der vorangehenden Ansprüche, wobei eine Haftklebstoffbeschichtung als Klebstoffbeschichtung (16) vorgesehen ist.

19. Spritzenanordnung gemäß einem der vorangehenden Ansprüche, wobei die unterseitig nichtklebenden Bereiche des Etiketts durch Aussparungen in der Klebstoffbeschichtung (16) gebildet sind.

20. Spritzenanordnung gemäß einem der Ansprüche 1-19, wobei die unterseitig nichtklebenden Bereiche (26a, 26b) des Etiketts durch lokale Neutralisierung einer sich über die gesamte Unterseite des Etiketts erstreckenden Klebstoffbeschichtung (16) gebildet sind.

21. Spritzenanordnung gemäß einem der vorangehenden Ansprüche, wobei die Trennlinie bzw. Trennlinien (18a, 18b, 18c) jeweils als Perforationslinie oder Anstanzung ausgebildet ist bzw. sind.

22. Spritzenanordnung gemäß einem der vorangehenden Ansprüche, wobei das Etikett einen Aufdruck in Form von Zahlen- und/oder Text- und/oder Bildinformation aufweist.

23. Spritzenanordnung gemäß einem der vorangehenden Ansprüche, wobei das Etikett teilweise transparent ist.

24. Spritzenanordnung gemäß einem der vorangehenden Ansprüche, wobei
- das Etikett an einem zylindrischen oder prismatischen Bereich des Spritzenkörpers (1) angebracht ist,
- die Ausdehnung des Grundabschnitts (11) in Streifenlängsrichtung größer ist als 50 Prozent des Umfangs des Spritzenkörpers (1) in dem zylindrischen oder prismatischen Bereich, und
- die Ausdehnung des gesamten Etiketts in Streifenlängsrichtung größer ist als der Umfang des Spritzenkörpers (1) in dem zylindrischen oder prismatischen Bereich.

25. Spritzenanordnung gemäß Anspruch 24, wobei die Ausdehnung des Grundabschnitts (11) in Streifenlängsrichtung größer ist als 90 Prozent des Umfangs des Spritzenkörpers (1) in dem zylindrischen oder prismatischen Bereich.

26. Spritzenanordnung gemäß einem der Ansprüche 24-25, wobei die Ausdehnung des gesamten Etiketts in Streifenlängsrichtung größer ist als das Zweifache des Umfangs des Spritzenkörpers (1) in dem zylindrischen oder prismatischen Bereich.

27. Spritzenanordnung gemäß Anspruch 26, wobei die Ausdehnung des Grundabschnitts (11) in Streifenlängsrichtung größer ist als der Umfang des Spritzenkörpers (1) in dem zylindrischen oder prismatischen Bereich.

28. Spritzenanordnung gemäß einem der Ansprüche 24-27, wobei das Etikett an dem zylindrischen oder prismatischen Bereich des Spritzenkörpers (1) sich selbst überlappend so angebracht ist, daß
- die Klebstoffbeschichtung (16) des Grundabschnitts (11) an dem Spritzenkörper (1) haftet,
- der Belegabschnitt bzw. die Belegabschnitte (12a, 12b) unterseitig zumindest überwiegend auf der klebstoffabweisenden Oberfläche aufliegen, und der Schutzabschnitt (13) die Belegabschnitte (12a, 12b) überdeckt.

## Claims

1. A syringe assembly, comprising:
- an external body formed as a syringe protector and comprising a receptacle (4) having at least one lateral opening (24) and an insert (5) having at least one lateral opening (25),
- an internal body which is formed as a syringe body (1) and inserted in the syringe protector, the syringe body (1) being provided with a label,
wherein the label in the longitudinal direction of the strip comprises the following portions next to one another:
- at least one document portion (12a, 12b) which in the longitudinal direction of the strip can be severed from the remainder of the label along at least one dividing line (18a, 18b, 18c) extending in the transverse direction of the strip and has its underside provided with an adhesive coating (16) at least in portions,
- a grip tab (15) which has its underside designed so as to be non-adhesive and, as seen from the at least one document portion (12a, 12b), is terminally arranged on the label in the longitudinal direction of the strip,
- a base portion (11) which has its upper side provided with an adhesive-repellent surface at least in portions and has its underside provided with an adhesive coating (16) at least in portions,
wherein the label, by pulling the grip tab (15), can be partially unrolled from the syringe body (1) through the lateral openings (24, 25) of the insert (5) and the receptacle (4) and can be pulled out of the syringe protector, so that the document portions (12a, 12b) can be severed without having to take out the syringe body (1) from the syringe protector.

2. The syringe assembly according to claim 1, wherein the base portion (11) in the longitudinal direction of the strip is arranged next to the document portion(s) (12a, 12b), the document portion(s) (12a, 12b) can be severed at both sides from the remainder of the label along dividing lines (18a, 18b, 18c) which extend in the transverse direction of the strip, and the grip tab (15) in the longitudinal direction of the strip is arranged on the side of the label opposite to the base portion (11) .

3. The syringe assembly according to claim 1, wherein the base portion (11) is arranged underneath the document portion(s) (12a, 12b), and the adhesive coating (16) of the document portion(s) (12a, 12b) adhere to the adhesive-repellent surface of the base portion (11).

4. The syringe assembly according to claim 3, comprising an initial portion (26) which in the longitudinal direction of the strip is terminally arranged on the side of the label opposite the grip tab (15).

5. The syringe assembly according to any of the preceding claims, wherein the label further comprises
- a protective portion (13) which adjoins a document portion (12b) on one side and has its underside designed so as to be non-adhesive at least for the major part of its surface area,
- a retaining portion (14) which in the longitudinal direction of the strip adjoins the protective portion (13) at the side opposite the document portion(s) (12a, 12b) and has its underside provided with an adhesive coating (16) at least in portions,
wherein the grip tab (15) in the longitudinal direction of the strip adjoins the retaining portion (14) at the side opposite the protective portion (13).

6. The syringe assembly according to claim 5, wherein the adhesive-repellent surface and the protective portion (13) each have at least the same extension in the longitudinal direction of the strip as the document portion or the entirety of the document portions (12a, 12b).

7. The syringe assembly according to any of the claims 5-6, wherein the extension of the retaining portion (14) in the longitudinal direction of the strip is smaller than the extension of the protective portion (13).

8. The syringe assembly according to any of the preceding claims, wherein the extension of the grip tab (15) in the longitudinal direction of the strip continuously increases in the transverse direction of the strip across a part of the strip width.

9. The syringe assembly according to claim 8, wherein the extension of the grip tab (15) in the longitudinal direction of the strip continuously increases in the transverse direction of the strip across the major part of the strip width.

10. The syringe assembly according to any of the claims 8-9, wherein the smallest extension of the grip tab (15) in the longitudinal direction of the strip amounts to between 0 and 25 percent of the largest extension of the grip tab (15) in the longitudinal direction of the strip.

11. The syringe assembly according to claim 10, wherein the smallest extension of the grip tab (15) in the longitudinal direction of the strip amounts to between 0 and 10 percent of the largest extension of the grip tab (15) in the longitudinal direction of the strip.

12. The syringe assembly according to any of the preceding claims, wherein the largest extension of the grip tab (15) in the longitudinal direction of the strip amounts to at least 3 percent of the extension of the base portion (11) in the longitudinal direction of the strip.

13. The syringe assembly according to claim 12, wherein the largest extension of the grip tab (15) in the longitudinal direction of the strip amounts to at least 5 percent of the extension of the base portion (11) in the longitudinal direction of the strip.

14. The syringe assembly according to any of the preceding claims, wherein the largest extension of the grip tab (15) in the longitudinal direction of the strip is at least 1 mm.

15. The syringe assembly according to claim 14, wherein the largest extension of the grip tab (15) in the longitudinal direction of the strip is at least 2.5 mm.

16. The syringe assembly according to any of the preceding claims, wherein the dividing line(s) (18a, 18b, 18c) extend(s) in the transverse direction of the strip across the entire label.

17. The syringe assembly according to any of the preceding claims, wherein the adhesive-repellent surface is produced by siliconization (17).

18. The syringe assembly according to any of the preceding claims, wherein a pressure-sensitive adhesive coating is provided as the adhesive coating (16).

19. The syringe assembly according to any of the preceding claims, wherein the non-adhesive areas of the label on the underside thereof are formed by recesses in the adhesive coating (16) .

20. The syringe assembly according to any of the claims 1-19, wherein the non-adhesive areas (26a, 26b) of the label on the underside thereof are formed by a local neutralization of an adhesive coating (16) extending across the entire underside of the label.

21. The syringe assembly according to any of the preceding claims, wherein the dividing line(s) (18a, 18b, 18c) is/are each formed as a perforation line or as a stamping.

22. The syringe assembly according to any of the preceding claims, wherein the label comprises an imprint in the form of numerical and/or textual and/or image information.

23. The syringe assembly according to any of the preceding claims, wherein the label is transparent at least in parts.

24. The syringe assembly according to any of the preceding claims, wherein
- the label is attached to a cylindrical or prismatic area of the syringe body (1),
- the extension of the base portion (11) in the longitudinal direction of the strip is larger than 50 percent of the circumference of the syringe body (1) in the cylindrical or prismatic area, and
- the extension of the entire label in the longitudinal direction of the strip is larger than the circumference of the syringe body (1) in the cylindrical or prismatic area.

25. The syringe assembly according to claim 24, wherein the extension of the base portion (11) in the longitudinal direction of the strip is larger than 90 percent of the circumference of the syringe body (1) in the cylindrical or prismatic area.

26. The syringe assembly according to any of the claims 24-25, wherein the extension of the entire label in the longitudinal direction of the strip is larger than twice the circumference of the syringe body (1) in the cylindrical or prismatic area.

27. The syringe assembly according to claim 26, wherein the extension of the base portion (11) in the longitudinal direction of the strip is larger than the circumference of the syringe body (1) in the cylindrical or prismatic area.

28. The syringe assembly according to any of the claims 24-27, wherein the label is attached to the cylindrical or prismatic area of the syringe body (1) in self-overlapping manner such that
- the adhesive coating (16) of the base portion (11) adheres to the syringe body (1),
- the underside of the document portion(s) (12a, 12b) rests on the adhesive-repellent surface at least for the major part, and the protective portion (13) covers the document portions (12a, 12b).

## Revendications

1. Agencement de seringue, présentant :
- un corps extérieur constitué comme protecteur de seringue, comportant un logement (4) présentant au moins un orifice latéral (24) et un insert (5) présentant au moins un orifice latéral (25),
- un corps intérieur constitué comme corps de seringue (1) et qui est inséré dans le protecteur de seringue, sachant que le corps de seringue (1) est pourvu d'une étiquette, sachant que l'étiquette présente des sections qui se suivent les unes à côté des autres en direction longitudinale de bande :
- au moins une section de justificatif (12a, 12b), laquelle est détachable de l'étiquette restante en direction longitudinale de bande le long d'au moins une ligne de séparation (18a, 18b, 18c) s'étendant en direction transversale de bande et présente en face inférieure au moins partiellement un revêtement adhésif (16),
- une languette de prise (15), laquelle est exécutée de manière non adhésive en face inférieure, et est disposée sur l'étiquette en position finale en direction longidutinale de bande en regardant depuis l'au moins une section de justificatif (12a, 12b),
- une section de base (11), laquelle présente en face supérieure au moins partiellement une surface anti-adhésive et en face inférieure au moins partiellement un revêtement adhésif (16),
sachant que l'étiquette peut être déroulée en partie du corps de seringue (1) à travers les orifices latéraux (24, 25) de l'insert (5) ou du logement (4) en tirant sur la languette de prise (15) et peut être extraite du protecteur de seringue de sorte que les sections de justificatif (12a, 12b) puissent être détachées sans devoir ôter le corps de seringue (1) du protecteur de seringue.

2. Agencement de seringue selon la revendication 1, sachant que la section de base (11) est disposée à côté de la section de justificatif / des sections de justificatif (12a, 12b) en direction longitudinale de bande, la section de justificatif / les sections de justificatif (12a, 12b) est / sont détachable/s de l'étiquette restante des deux côtés le long de lignes de séparation (18a, 18b, 18c) s'étendant en direction transversale de bande, et la languette de prise (15) est disposée du côté de l'étiquette qui est opposé à la section de base (11) en direction longitudinale de bande.

3. Agencement de seringue selon la revendication 1, sachant que la section de base (11) est disposée sous la section de justificatif / les sections de justificatif (12a, 12b), et le revêtement adhésif (16) de la section de justificatif / des sections de justificatif (12a, 12b) adhère à la surface anti-adhésive de la section de base (11).

4. Agencement de seringue selon la revendication 3, lequel présente une section initiale (26), laquelle est disposée en position finale du côté de l'étiquette qui est opposé à la languette de prise (15) en direction longitudinale de bande.

5. Agencement de seringue selon l'une des revendications précédentes, sachant que l'étiquette présente en outre
- une section de protection (13), laquelle est d'un côté limitrophe d'une section de justificatif (12b) et est exécutée en face inférieure de manière non adhésive au moins pour l'essentiel de sa surface,
- une section de maintien (14), laquelle est contiguë à la section de protection du côté opposé à la section de justificatif / aux sections de justificatif (12a, 12b) en direction longitudinale de bande et présente en face inférieure au moins partiellement un revêtement adhésif (16),
sachant que la languette de prise (15) est contiguë à la section de maintien (14) du côté opposé à la section de protection (13) en direction longitudinale de bande.

6. Agencement de seringue selon la revendication 5, sachant que la surface anti-adhésive et la section de protection (13) ont chacune en direction longitudinale de bande au moins le même allongement que la section de justificatif / la totalité des sections de justificatif (12a, 12b).

7. Agencement de seringue selon l'une des revendications 5 à 6, sachant que l'allongement de la section de maintien (14) en direction longitudinale de bande est moindre que l'allongement de la section de protection (13).

8. Agencement de seringue selon l'une des revendications précédentes, sachant que l'allongement de la languette de prise (15) en direction longitudinale de bande augmente continuellement sur une partie de la largeur de bande en direction transversale de bande.

9. Agencement de seringue selon la revendication 8, sachant que l'allongement de la languette de prise (15) en direction longitudinale de bande augmente continuellement sur la majeure partie de la largeur de bande en direction transversale de bande.

10. Agencement de seringue selon l'une des revendications 8 à 9, sachant que le plus petit allongement de la languette de prise (15) en direction longitudinale de bande est compris entre 0 et 25 pour cent du plus grand allongement de la languette de prise (15) en direction longitudinale de bande.

11. Agencement de seringue selon la revendication 10, sachant que le plus petit allongement de la languette de prise (15) en direction longitudinale de bande est compris entre 0 et 10 pour cent du plus grand allongement de la languette de prise (15) en direction longitudinale de bande.

12. Agencement de seringue selon l'une des revendications précédentes, sachant que le plus grand allongement de la languette de prise (15) en direction longitudinale de bande est d'au moins 3 pour cent de l'allongement de la section de base (11) en direction longitudinale de bande.

13. Agencement de seringue selon la revendication 12, sachant que le plus grand allongement de la languette de prise (15) en direction longitudinale de bande est d'au moins 5 pour cent de l'allongement de la section de base (11) en direction longitudinale de bande.

14. Agencement de seringue selon l'une des revendications précédentes, sachant que le plus grand allongement de la languette de prise (15) en direction longitudinale de bande est d'au moins 1 mm.

15. Agencement de seringue selon la revendication 14, sachant que le plus grand allongement de la languette de prise (15) en direction longitudinale de bande est d'au moins 2,5 mm.

16. Agencement de seringue selon l'une des revendications précédentes, sachant que la ligne de séparation / les lignes de séparation (18a, 18b, 18c) s'étend / s'étendent sur toute l'étiquette en direction transversale de bande.

17. Agencement de seringue selon l'une des revendications précédentes, sachant que la surface anti-adhésive est formée par un siliconage (17).

18. Agencement de seringue selon l'une des revendications précédentes, sachant qu'un revêtement adhésif cohésif est prévu comme revêtement adhésif (16).

19. Agencement de seringue selon l'une des revendications précédentes, sachant que les zones de l'étiquette non adhésives en face inférieure sont formées par des évidements dans le revêtement adhésif (16).

20. Agencement de seringue selon l'une des revendications 1 à 19, sachant que les zones (26a, 26b) de l'étiquette non adhésives en face inférieure sont formées par neutralisation locale d'un revêtement adhésif (16) qui s'étend sur toute la face inférieure de l'étiquette.

21. Agencement de seringue selon l'une des revendications précédentes, sachant que la ligne de séparation / les lignes de séparation (187a, 18b, 18c) est / sont constituée/s chacune comme ligne de perforation ou poinçonnage.

22. Agencement de seringue selon l'une des revendications précédentes, sachant que l'étiquette présente une impression sous forme d'informations de chiffres et/ou de texte et/ou d'image.

23. Agencement de seringue selon l'une des revendications précédentes, sachant que l'étiquette est en partie transparente.

24. Agencement de seringue selon l'une des revendications précédentes, sachant que
- l'étiquette est apposée sur une zone cylindrique ou prismatique du corps de seringue (1),
- l'allongement de la section de base (11) en direction longitudinale de bande est supérieur à 50 pour cent de la circonférence du corps de seringue (1) dans la zone cylindrique ou prismatique, et
- l'allongement de toute l'étiquette en direction longitudinale de bande est supérieur à la circonférence du corps de seringue (1) dans la zone cylindrique ou prismatique.

25. Agencement de seringue selon la revendication 24, sachant que l'allongement de la section de base (11) en direction longitudinale de bande est supérieur à 90 pour cent de la circonférence du corps de seringue (1) dans la zone cylindrique ou prismatique.

26. Agencement de seringue selon l'une des revendications 24 à 25, sachant que l'allongement de l'ensemble de l'étiquette en direction longitudinale de bande est supérieur au double de la circonférence du corps de seringue (1) dans la zone cylindrique ou prismatique.

27. Agencement de seringue selon la revendication 26, sachant que l'allongement de la section de base (11) en direction longitudinale de bande est supérieur à la circonférence du corps de seringue (1) dans la zone cylindrique ou prismatique.

28. Agencement de seringue selon l'une des revendications 24 à 27, sachant que l'étiquette est apposée de manière auto-chevauchante sur la zone cylindrique ou prismatique du corps de seringue (1) de telle façon que
- le revêtement adhésif (16) de la section de base (11) adhère au corps de seringue (1),
- la section de justificatif / les sections de justificatif (12a, 12b) repose / reposent en face inférieure au moins pour l'essentiel sur la surface anti-adhésive, et la section de protection (13) recouvre les sections de justificatif (12a, 12b).
